# EUROPÄISCHE PATENTSCHRIFT

(11) **EP 0 632 025 B1**
(45) Veröffentlichungstag und Bekanntmachung des Hinweises auf die Patenterteilung: **25.10.2000**
(21) Anmeldenummer: 94109544.0
(22) Anmeldetag: 21.06.1994
(51) Int. Cl.: C07D 213/61, C07D 213/73, C07D 213/26, C07D 213/38

(54) **Verfahren zur Herstellung von 2-Chlor-5-chlormethylpyridin**
Process for the preparation of 2-chloro-5-chloromethylpyridine
Procédé pour la préparation de 2-chloro-5-chlorométhylpyridine

(30) Priorität: 02.07.1993 DE 4322054
(43) Veröffentlichungstag der Anmeldung: 04.01.1995
(73) Patentinhaber: BAYER AG, 51368 Leverkusen (DE)
(72) Erfinder: Kraus, Helmut, Dr., D-51519 Odenthal (DE); Lindel, Hans, Dr., D-51373 Leverkusen (DE); Diehr, Hans-Joachim, Dr., D-42329 Wuppertal (DE)

(56) Entgegenhaltungen:
- EP-A- 0 508 215
- JP-A- 5 178 835
- RECUEIL DES TRAVAUX CHIMIQUES DES PAYS-BAS Bd. 55, Nr. 17 , 1936 , AMSTERDAM Seiten 293 - 296 OVERHOFF J ET AL. 'Das 2-(2-pyridyl)-alanin'
- CHEMISCHES CENTRAL-BLATT Bd. II, Nr. 16 , 19. Oktober 1898 Seiten 887 - 888 SOLONINA W. 'Zur Einwirkung von Nitrosylchlorid auf Amine der Fettreihe. I. Einwirkung von NOCl auf primäre Monamine'
- CHEMICAL REVIEWS Bd. 48 , 1951 Seiten 319 - 396 BECKHAM LJ ET AL. 'Nitrosylchloride'
- ANGEWANDTE CHEMIE Bd. 75, Nr. 5 , 7. März 1963 , WEINHEIM Seiten 235 - 240 MATHES W & SCHÜLY H 'In der Seitenkette halogenierte Methylpyridine und Methylchinoline'
- BAKKE J.M.: "Acid-catalyzed formation of diazoalkanes. A one-pot transformation of alkylamines to diazoalkanes", ACTA CHEMICA SCANDINAVICA, , 1982, Band B36, Nr. 2, Seiten 127 - 129

## Beschreibung

Die Erfindung betrifft ein neues Verfahren zur Herstellung von 2-Chlor-5-chlormethylpyridin.

Es ist bekannt, dass Chlormethylpyridine durch Umsetzung von Hydroxymethylpyridinen mit Thionylchlorid erhalten werden können (vgl. J. Heterocycl. Chem. 16 (1979), 333-337; vgl. auch EP-A 373 464).

Weiter ist bekannt, dass man Chlormethylpyridine erhält, wenn man Methylpyridine in Gegenwart von Säureakzeptoren oder Radikalbildnern und in Gegenwart von inerten Verdünnungsmitteln bei Temperaturen zwischen 0°C und 100°C chloriert (vgl. EP-A 260 485, vgl. auch EP-A 458 109).

Ferner ist bekannt, dass auch Alkoxymethylpyridine durch Umsetzung mit geeigneten Chlorierungsmitteln in Chlormethylpyridine umgewandelt werden können (vgl. EP-A 393 453).

Es ist auch bekannt, dass Benzylamin durch Umsetzung mit Nitrosylchlorid in Benzylchlorid umgewandelt werden kann (Chem. Zentralblatt, 16, 887, 1898).

Es wurde nun gefunden, dass man 2-Chlor-5-chlormethylpyridin der Formel (I) 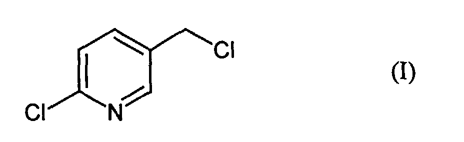 in guten Ausbeuten erhält, wenn man 2-Chlor-5-aminomethylpyridin der Formel (II) 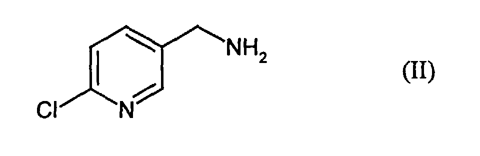 mit Nitrosylchlorid in Gegenwart eines organischen Verdünnungsmittels bei Temperaturen zwischen -10°C bis +40°C umsetzt und dann auf übliche Weise aufarbeitet.

Überraschenderweise kann nach dem erfindungsgemäßen Verfahren 2-Chlor-5-chlormethylpyridin der Formel (I) in guten Ausbeuten erhalten werden.

Der Ausgangsstoff der Formel (II) ist bekannt und/oder kann nach an sich bekannten Verfahren hergestellt werden (vgl. DE-OS 37 26 993 und WO 92/13840).

Das erfindungsgemäße Verfahren wird in Gegenwart eines Verdünnungsmittels durchgeführt. Als Verdünnungsmittel kommen die üblichen organischen Solventien in Betracht, beispielsweise (gegebenenfalls chlorierte) Kohlenwasserstoffe wie Pentan, Hexan, Heptan, Octan, Cyclohexan, Methylcyclohexan, Petrolether, Benzin, Ligroin, Methylenchlorid, Ethylenchlorid, Chloroform, Tetrachlormethan, Chlorbenzol und o-Dichlorbenzol, Ether wie Diethylether, Dipropylether, Diisopropylether, Dibutylether, Diisobutylether, Methyl-tert-butylether, Methyl-tert-pentylether, Ethylenglycoldimethylether, Tetrahydrofuran und Dioxan, Ketone wie Aceton, Diethylketon, Methylisopropylketon und Methyl-isobutyl-keton, Ester wie Essigsäure-methylester und Essigsäure-ethylester, Nitrile wie Acetonitril und Propionitril, Amide wie N,N-Dimethyl-formamid, N,N-Diethyl-formamid, N,N-Dipropyl-formamid, N,N-Dibutyl-formamid, N,N-Dicyclohexyl-formamid und N,N-Dimethyl-acetamid, ferner auch N-Methyl-pyrrolidon, Tetramethylharnstoff, Dimethylsulfoxid, Tetramethylensulfon, flüssiges Schwefeldioxid und Wasser.

Es können auch Zweiphasensysteme aus Wasser und den oben angegebenen organischen Lösungsmitteln, soweit mit Wasser praktisch nicht mischbar - als Verdünnungsmittel verwendet werden, wobei dann vorzugsweise Phasentransfer-Katalysatoren eingesetzt werden. Als Beispiele für solche Katalysatoren seien genannt: Tetrabutylammoniumbromid, Tetrabutylammoniumchlorid, Tetraoctylammoniumchlorid, Tetrabutylammoniumhydrogensulfat, Methyltrioctylammoniumchlorid, Hexadecyltrimethylammoniumchlorid, Hexadecyltrimethylammoniumbromid, Benzyltrimethylammoniumchlorid, Benzyltriethylammoniumchlorid, Benzyl-trimethylammoniumhydroxid, Benzyltriethylammoniumhydroxid, Benzyltributylammoniumchlorid, Benzyltributylammoniumbromid, Tetrabutylphosphoniumbromid, Tetrabutylphosphoniumchlorid, Tributylhexadecylphosphoniumbromid, Butyltriphenylphosphoniumchlorid, Ethyltrioctylphosphoniumbromid, Tetraphenylphosphoniumbromid.

Die Reaktionstemperaturen können bei dem erfindungsgemäßen Verfahren in einem größeren Bereich variiert werden. Im allgemeinen arbeitet man bei Temperaturen zwischen -10°C und +40°C.

Das erfindungsgemäße Verfahren wird im allgemeinen bei Normaldruck oder bei leicht erhöhtem oder leicht vermindertem Druck - im allgemeinen zwischen 0,1 und 10 bar - durchgeführt.

Zur Durchführung des erfindungsgemäßen Verfahrens setzt man auf 1 Mol der Verbindung der Formel (II) im allgemeinen zwischen 1 und 5 Mol, vorzugsweise zwischen 2 und 3 Mol Nitrosylchlorid ein.

In einer bevorzugten Ausführungsform des erfindungsgemäßen Verfahrens wird die Verbindung der Formel (II) in einem der oben angegebenen Verdünnungsmittel vorgelegt und eine Lösung von Nitrosylchlorid in einem der oben angegebenen Verdünnungsmittel wird unter Rühren eindosiert. Das Reaktionsgemisch wird dann bis zum Ende der Umsetzung gerührt; anschließend wird der Ansatz auf übliche Weise aufgearbeitet.

In einer weiteren bevorzugten Ausführungsform des erfindungsgemäßen Verfahrens wird Nitrosylchlorid in einem der oben angegebenen Verdünnungsmittel vorgelegt und eine Lösung der Verbindung der Formel (II) in einem der oben angegebenen Verdünnungsmittel wird unter Rühren eindosiert. Das Reaktionsgemisch wird dann bis zum Ende der Umsetzung gerührt; anschließend wird der Ansatz auf übliche Weise aufgearbeitet.

Die Aufarbeitung kann nach üblichen Methoden durchgeführt werden. Beispielsweise wird das Reaktionsgemisch - gegebenenfalls nach Einengen - mit Wasser verdünnt, mit Natronlauge oder Natrium-(hydrogen)carbonat neutralisiert und die organische Phase gegebenenfalls mit einem mit Wasser praktisch nicht mischbaren organischen Lösungsmittel, wie z.B. Methylenchlorid verdünnt. Nach Durchschütteln wird die organische Phase abgetrennt, getrocknet und filtriert. Vom Filtrat wird dann das organische Lösungsmittel unter vermindertem Druck sorgfältig abdestilliert, wobei man das Produkt der Formel (I) als öligen Rückstand erhält, welcher nach üblichen Methoden weiter gereinigt werden kann.

Die nach dem erfindungsgemäßen Verfahren herzustellende Verbindung der Formel (I) kann als Zwischenprodukt zur Herstellung von biologisch aktiven Verbindungen, beispielsweise von Insektiziden verwendet werden (vgl. EP-A 163855 und EP-A 192060).

### Herstellungsbeispiele

### Beispiel 1

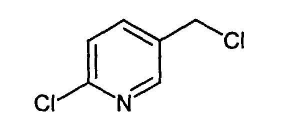

Eine Lösung von 9,2 g (141 mmol) Nitrosylchlorid in 50 ml Methylenchlorid wird bei 10°C innerhalb von ca. 90 Minuten unter Rühren zu einer Lösung von 10,0 g (70 mmol) 2-Chlor-5-aminomethyl-pyridin in 200 ml Methylenchlorid tropfenweise gegeben. Das Reaktionsgemisch wird dann noch 2 Stunden bei 20°C gerührt. Anschließend werden 100 ml Wasser dazu gegeben und durch Zugabe von Natriumhydrogencarbonat wird der pH-Wert auf 7 eingestellt. Die organische Phase wird dann abgetrennt und die wässrige Phase noch zweimal mit je 50 ml Methylenchlorid nachextrahiert. Von den vereinigten organischen Phasen wird dann das Lösungsmittel im Wasserstrahlvakuum sorgfältig abdestilliert.

Der verbleibende Rückstand enthält nach gaschromatographischer Analyse 6,8 g (60 % der Theorie) 2-Chlor-5-chlormethylpyridin und 3,5 g (35 % der Theorie) 2-Chlor-5-hydroxymethyl-pyridin.

Das als Nebenprodukt erhaltene 2-Chlor-5-hydroxymethyl-pyridin kann nach üblichen Methoden abgetrennt und auf bekannte Weise (vgl. EP-A 373 464) in 2-Chlor-5-chlormethyl-pyridin umgewandelt werden.

### Beispiel 2

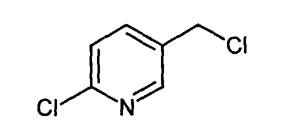

25 ml N,N-Dimethyl-formamid werden bei 20°C mit Hydrogenchlorid gesättigt. Diese Lösung wird mit weiteren 200 ml N,N-Dimethyl-formamid verdünnt und nach Abkühlen auf -10°C werden 20 ml vorgekühltes Nitrosylchlorid dazu gegeben. Man lässt die Mischung auf 0°C kommen und gibt bei dieser Temperatur eine Lösung von 17,3 g (122 mmol) 2-Amino-5-aminomethyl-pyridin in 100 ml N,N-Dimethylformamid tropfenweise dazu. Dann lässt man das Reaktionsgemisch auf Raumtemperatur (20°C) kommen und rührt es bei dieser Temperatur noch eine Stunde. Anschließend wird es im Wasserstrahlvakuum eingeengt, durch Zugabe von 50 ml wässriger 0,2 N-Natronlauge neutralisiert und mit 150 ml Methylenchlorid geschüttelt. Die organische Phase wird abgetrennt, mit Natriumsulfat getrocknet und filtriert. Vom Filtrat wird das Lösungsmittel im Wasserstrahlvakuum sorgfältig abdestilliert.

Man erhält 14 g eines Öls, das laut ¹H-NMR-Spektrum 85 % 2-Chlor-5-chlormethylpyridin (60 % d. Th.) enthält.

## Patentansprüche

1. Verfahren zur Herstellung von 2-Chlor-5-chlormethylpyridin der Formel (I) 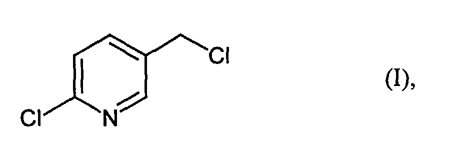 dadurch gekennzeichnet, dass man 2-Chlor-5-aminomethylpyridin der Formel 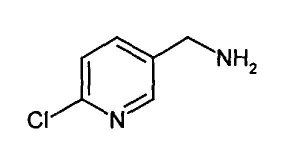 mit Nitrosylchlorid in Gegenwart eines organischen Verdünnungsmittels bei Temperaturen von -10°C bis 40°C umsetzt.

2. Verfahren zur Herstellung von 2-Chlor-5-chlormethylpyridin der Formel (I) gemäß Anspruch 1, dadurch gekennzeichnet, dass man 2-Chlor-5-aminomethylpyridin der Formel 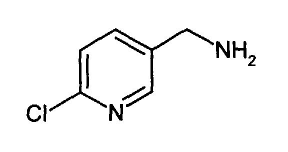 mit Nitrosylchlorid in Methylenchlorid bei Temperaturen von 10°C bis 20°C umsetzt.

3. Verfahren zur Herstellung von 2-Chlor-5-chlormethylpyridin der Formel (I) gemäß Anspruch 1, dadurch gekennzeichnet, dass man 2-Chlor-5-aminomethylpyridin der Formel 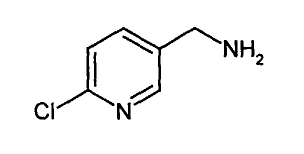 mit Nitrosylchlorid in Dimethylformamid bei Temperaturen von -10°C bis 20°C umsetzt.

## Claims

1. Process for preparing 2-chloro-5-chloromethylpyridine of the formula (I) 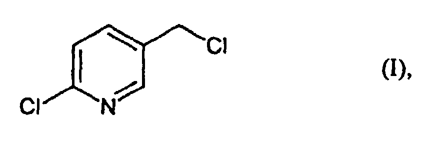 characterized in that 2-chloro-5-aminomethylpyridine of the formula 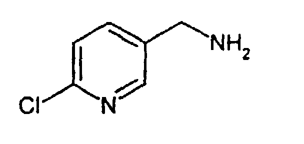 is reacted with nitrosyl chloride in the presence of an organic diluent at temperatures of from -10°C to 40°C.

2. Process for preparing 2-chloro-5-chloromethylpyridine of the formula (I) according to Claim 1, characterized in that 2-chloro-5-aminomethylpyridine of the formula 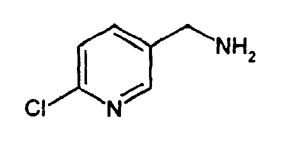 is reacted with nitrosyl chloride in methylene chloride at temperatures of from 10°C to 20°C.

3. Process for preparing 2-chloro-5-chloromethylpyridine of the formula (I) according to Claim 1, characterized in that 2-chloro-5-aminomethylpyridine of the formula 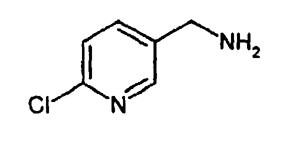 is reacted with nitrosyl chloride in dimethylformamide at temperatures of from -10°C to 20°C.

## Revendications

1. Procédé pour la préparation de la 2-chloro-5-chlorométhylpyridine répondant à la formule (I) 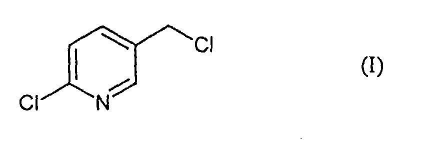 caractérisé en ce qu'on fait réagir de la 2-chloro-5-aminométhylpyridine répondant à la formule 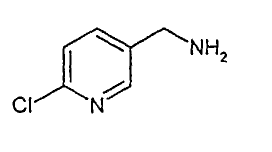 avec du chlorure de nitrosyle en présence d'un diluant organique à des températures de -10°C à 40°C.

2. Procédé pour la préparation de la 2-chloro-5-chlorométhylpyridine répondant à la formule (I) selon la revendication 1, caractérisé en ce qu'on fait réagir de la 2-chloro-5-aminométhylpyridine répondant à la formule 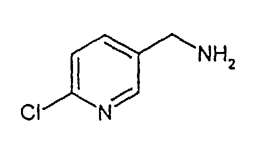 avec du chlorure de nitrosyle dans du chlorure de méthylène à des températures de 10°C à 20°C.

3. Procédé pour la préparation de la 2-chloro-5-chlorométhylpyridine répondant à la formule (I) selon la revendication 1, caractérisé en ce qu'on fait réagir de la 2-chloro-5-aminométhylpyridine répondant à la formule 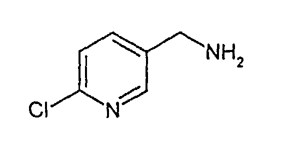 avec du chlorure de nitrosyle dans du diméthylformamide à des températures de -10°C à 20°C.
